# EUROPEAN PATENT SPECIFICATION

(11) **EP 3 349 701 B1**
(45) Date of publication and mention of the grant of the patent: **30.08.2023**
(21) Application number: 16766539.7
(22) Date of filing: 15.09.2016
(51) Int. Cl.: A61F 9/008, A61B 34/00

(54) **LASER ASSISTED EYE TREATMENT SYSTEM**
LASERUNTERSTÜTZTES AUGENBEHANDLUNGSSYSTEM
SYSTÈME DE TRAITEMENT OCULAIRE ASSISTÉ PAR LASER

(30) Priority: 17.09.2015 DE 102015217847
(43) Date of publication of application: 25.07.2018
(73) Proprietor: Carl Zeiss Meditec AG, 07745 Jena (DE)
(72) Inventor: ANDREWS, Delbert Peter, 73447 Oberkochen (DE); RILL, Michael Stefan, 07751 Jena (DE); GRÄBER, Stefan, 82041 Deisenhofen (DE); WERTH, Julia, 81373 München (DE)
(74) Representative: Rößner, Ulrike
(86) International application number: PCT/EP2016/071813
(87) International publication number: WO 2017/046239

(56) References cited:
- EP-A1- 2 846 258
- WO-A1-2015/025039
- US-A1- 2014 114 296

## Description

The present invention relates to a laser assisted eye treatment system for carrying out an eye treatment workflow, the system comprising a laser treatment unit generating a pulsed laser beam, a characterization unit, a graphical user interface with a control panel, configured to communicate information required for the eye treatment workflow and a system controller.

Laser systems are widely used for assistance in characterization and therapy in today's eye surgery. A laser beam which is used for therapy may "cut" tissue of a patient's eye or another material in a patient's eye by photo disruption, remove tissue by ablation or modify tissue by heat effects. Examples for the most common use of laser systems in eye surgery are the correction of astigmatism and cataract surgery, where pulsed laser beams, mostly femtosecond laser beams, are applied.

For such laser assisted eye treatment methods it is very important to work according to a very high level of security as damage in the eye tissue due to user errors is usually not reversible. Thus eye surgery which is involving laser sequences for therapy reasons has to be very well planned.

At the same time, a workflow for an eye surgery system involving laser therapy sequences is very complex. In particular, there are strong interactions between sequences of imaging of the eye in two dimensions, e.g. by video, and in three dimensions, e.g. by optical coherence tomography (OCT), a sequence of determination of the anatomical structures, a sequence of planning of the laser incisions in relationship to the anatomical structures and a sequence of presentation of the planned laser incisions in relationship to the anatomical structures.

In a laser assisted eye treatment system, much information required for an eye treatment workflow has "to be given" to the system either from outside or from internal units of the system to enable a smooth workflow. Even if required information is already proposed by the system, the correctness of the data needs to be approved in most cases. For this purpose, a graphical user interface is applied as a general "communication means" with the system. The graphical user interface is a multifunctional communication means, which is, for example, capable of setting system operating parameters, processing user input, displaying gathered information such as images, diagrams and data of characterized structures, and displaying representations of planned or proposed interventions.

A graphical user interface which is organized in a complex way, giving access to different steps of the workflow at the same time or displaying parameters which are not needed in a distinct step, is very difficult to handle and bears a high potential of errors for the surgeon or another person handling the system. Further, it potentially extends the treatment time. In most cases the patient's eye is docked to the system, which is very inconvenient for the patient.

To minimize the risk, prior art systems therefore are usually handled by a dedicated person. In some of these systems it is even necessary to use a mouse or a trackball, which is critical in a surgery environment supposed to be aseptic: Therefore, this dedicated person handling the system is placed at distance from the surgery position.

US 2014/0114296 A1 discloses a graphical user interface and a workflow for a laser assisted eye surgery system. The disclosed laser assisted eye surgery system comprises a single graphical user interface, including a large (touch screen) control panel. The laser assisted eye surgery system also comprises other user interfaces such as a laser footswitch, a docking control keypad, a patient interface RFID reader, an emergency laser stop button, a key switch and a patient chair joystick control. But all these other user interfaces do not allow to display data and images as well as a complex workflow so as to give access to these information for an active communication with the laser eye surgery system, including choosing data out of a variety of data, choosing a special workflow out of a proposal of alternatives, enter, evaluate and modify data. That's why only a graphical user interface may be used for guidance through the whole workflow of an eye surgery treatment. Such an eye surgery treatment being a complex process, there are sequences of the workflow requiring a large quantity of information being displayed at once and making the handling sometimes confusing. However, especially during the critical laser treatment sequences it is important to display "easy screens", which prevent from a wrong handling at a maximum.

**The problem** to be solved by the present invention is therefore to provide a laser assisted eye treatment system which may be operated safely and autonomously, especially during the critical sequences, by the surgeon via a simple comprehensible and intuitive structure and a respective method. It should be easily operable in a sterile environment, and actively avoid accidents arising from maloperation by the surgeon or another person handling the system. Further it should help to shorten the critical treatment period, e.g., if docking is used, to shorten the docking time of the eye to be treated by the laser assisted eye treatment system.

The problem is solved by a laser assisted eye treatment system for carrying out an eye treatment workflow. An eye treatment using a laser beam is a common method in ophthalmology: The laser beam might be used to "cut" the eye tissue or an artificial eye material, like an intraocular lens (IOL) by photodisruption. It also might be used to "bond" eye tissue together or to simply modify the eye tissue by coagulation. Further it might be used to modify the shape of a part of the eye by laser ablation.

Preferentially the laser assisted eye treatment system is used for cataract surgery as well as for refractive corrections. In General, an eye treatment using a laser beam requires a complex workflow to be carry out on a patient's eye.

The laser assisted eye treatment system comprises a laser treatment unit with a laser source. The laser source is generating a pulsed laser beam. Preferentially a femtosecond laser source, especially an infrared femtosecond laser source, or a picosecond laser source is applied. The laser beam may then be guided to a laser applicator which is advantageously part of the laser treatment unit.

The pulsed laser beam may be focused to a position in the eye to be treated by focusing optics. The focus of the pulsed laser beam may be displaced by a three-dimensional focus shifting system. Such a three-dimensional focus shifting system is preferentially comprising scanners that allow to displace the focus of the pulsed laser beam within a three dimensional treatment volume.

WO 2015/025039 A1 discloses the control of a high intensity focused ultrasound medical treatment system. The disclosed treatment system comprises two graphical user interfaces, one for a physician and on for a technician. The two users (physician and technician) may simultaneously edit parameters and operate the treatment system by their individual control panels. Typically, the physician provides guiding when the technician creates a plan for the treatment system. The two users may control each other. It is thus a system to be run by both the physician and the technician simultaneously.

EP 2 846 258 A1 discloses a method for guiding user input to a computer system of an analysis system for processing biological samples. The computer system is directed to guide the user input especially of less trained persons. Therefore, different applications, that are executable on a processor and displayed on a display screen, are disclosed, that allow variable access to a given set of graphical user interface elements arranged in a predetermined order on that same display screen.

The laser assisted eye treatment system further comprises a characterization unit. The characterization unit is used to image structures of the patient's eye and may be applied before starting the eye treatment by the laser beam. It also may be used during the eye treatment to control its progress or after the eye treatment to verify the results. The characterization unit is preferentially used to measure data of a patient's eye, but also to allow an overview of the structures and the placement of the eye treatment.

The characterization unit may comprise a three-dimensional imaging and/or measurement system, especially an optical coherence tomography (OCT) system, an ultrasonic system, a Scheimpflug camera or an X-ray system. This system may be combined with a video or other optical imaging system which produces a 2D top view of the eye, such as an operation microscope.

Configured to communicate information required for the eye treatment workflow, the laser assisted eye treatment system also includes a first control panel comprising a first graphical user interface (GUI). The graphical user interface allows the user to interact with the laser assisted eye treatment system in very different ways, e.g., by entering data, displaying data, images and graphics, but also through graphical icons and visual indicators. The actions in a GUI may be performed through direct manipulation of the graphical elements and thus give the possibility of displaying graphics as well as text based information and data on the control panel display.

To "communicate" is used here as a generic term for different actions: It means to access data or information, especially parameters required for the treatment, and/or to choose data or information among a variety of different proposals. It may also include not only to use measured data or information but also to enter or to modify, i.e. to manipulate, data or information of the eye treatment system and/or of a patient's eye.

Last but not least the laser assisted eye treatment system comprises a system controller, configured to control the laser assisted eye treatment system. The controller may be realized as a single part controller or comprise several parts. The controller may modify at least one parameter in response to a user input. The controller therefore is operatively coupled to the first control panel providing the first graphical user interface as well as to the laser treatment unit and the characterization unit via a communication path. The first graphical user interface is thus encoded in the system controller for that means. Alternatively it can be transmitted to the system controller by a program storage means or via an internet connection.

If the controller comprises several parts, these parts may be physically located at very different positions, i.e. directly attached to the feature of the eye treatment system which is controlled by this part of the controller. The communication path also provides for the communication between the several parts of the control panel.

According to the invention the laser assisted eye treatment system comprises a second control panel comprising a second graphical user interface, configured to communicate a subset of information out of an entire set of information required for the eye treatment workflow. The subset is a restricted selection of information out of the entire set of information. This second graphical user interface is also encoded in the system controller for that means. Alternatively it can also be transmitted to the system controller by a program storage means or via an internet connection.

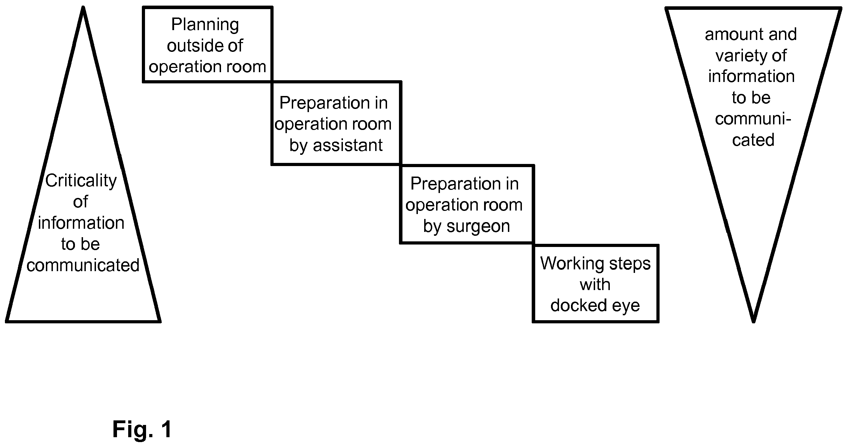

As schematically shown in fig. 1, there is a great amount and variety of information necessary for an eye treatment workflow that is considered not to be critical, as it might be prepared and communicated in advance before the eye treatment or after the eye treatment. It is not time critical and a correction of a given wrong information is generally possible. On the other hand, a small amount of information to be communicated, especially the information to be dealt with during the eye treatment itself, is considered very critical. An error within these information would be grievous.

While the first graphical user interface gives access to a large amount of data and information, in general to the entire set of information required for the possible eye treatment workflows that might be run on the laser assisted eye treatment system, the second graphical user interface gives access to a subset of the information and data concerning the eye treatment workflow only: It is the restricted selection of information out of the entire set of information which shall be directly available to the surgeon and which will be used during the laser treatment by him. All additional information is not communicated to the second graphical user interface: This avoids overloading of the second graphical user interface and enables the surgeon who is responsible for the eye treatment to personally have access to this critical information at all times.

Though they are generally situated at a distance to each other, the first graphical user interface displayed on the first control panel and second graphical user interface displayed on the second control panel are in correspondence to each other via the system controller and the communication path.

The second graphical user interface is an additional graphical user interface of the laser assisted eye treatment system, beside a first graphical user interface. It is therefore not just a copy of the first one. Additionally, the laser assisted eye treatment system may comprise further user interfaces such as foot switches or foot control panels, mice, trackballs etc., and automatic I/O devices with I/O interfaces, the latter ones receiving information or data automatically from units of the system or from outside to system to forward it to a special unit of the system, but none of these latter user interfaces is able to control an entire workflow.

In a special embodiment, the first graphical user interface of the laser assisted eye treatment system is configured to communicate a first subset of information and the second graphical user interface is configured to communicate a second subset of information out of the entire set of information required for the eye treatment workflow, wherein the first and the second subset of information are non-overlapping or partly overlapping subsets.

Non-overlapping subsets means that none of the information to be communicated out of the first subset is also part of the information to be communicated out of the second subset. Communicating an information via the first graphical user interface makes thus this same information unable to be communicated via the second graphical user interface. If, for instance, an eye treatment workflow is started on the first graphical user interface and the workflow is handed over to the second graphical user interface, there is no possibility to intervene via the first graphical user interface unless the workflow is handed back from the second graphical user interface to the first graphical user interface - either automatically, because predetermined in the workflow, or by the surgeon having finished or interrupted his part at the second graphical user interface. For this preferred special embodiment the communication of information via the first graphical user interface or the second graphical user interface always guarantees a clear responsibility without conflicts between a person working at the first graphical user interface and the person, generally the surgeon, working at the second graphical user interface.

Partly overlapping subsets as an alternative special embodiment of the laser assisted eye treatment system means that a part of the information out of the first subset is also part of the information out of the second subset of information. There is information that can be communicated via the first graphical user interface as well as via the second graphical user interface. This alternative embodiment may be used in situations where it is desired that an intervention by another person is possible via the first graphical user interface while the surgeon is working using the second graphical user interface to communicate information for the eye treatment workflow.

The management of the subsets may be done via the system controller.

In a further preferred embodiment of the laser assisted eye treatment system the eye treatment workflow comprises several working steps corresponding to different stages of an eye treatment process. Several working steps herein means at least two or more working steps, grouping the communication of a variety of data and other information belonging to a special area of communication.

Each of the working steps is then assigned to the first graphical user interface or to the second graphical user interface. For particular working steps, an assignment to both the first and the second graphical user interface is possible: Especially a last preparation working step before handling over the command to the surgeon at the second graphical user interface might be a working step that can be accessed by the first as well as be the second graphical user interface.

The ideal workflow of an laser assisted eye treatment system, especially of a laser assisted eye treatment system using a pulsed laser beam, is thus advantageously designed such that most working steps are done during the non-critical sequences and fewer working steps are done during the critical sequences of a laser assisted eye treatment process.

Using the first and the second graphical user interfaces of the laser assisted eye treatment system during an eye treatment by a laser beam at its highest merit, a working step or simply all communication of data which is not necessarily done during the critical sequences, for instance during the laser treatment working step or during all working steps where the patient's eye is docked to the laser assisted eye treatment system are not accessible via the second graphical user interface, but via the first graphical user interface. Vice versa, communication of information for a critical working step is not accessible via the first graphical user interface, but via the second graphical user interface, and thus always accessible to the surgeon.

Preferentially, the laser assisted eye treatment system further comprises a docking unit, which is arranged for establishing a defined relationship between the laser assisted eye treatment system and a patient's eye to be treated. Such a docking unit may imply the use of a liquid-filled patient interface or of a simple contact glass.

For an easy handling it is further very advantageous, if the second graphical user interface of the laser assisted eye treatment system and especially its second control panel is accessible to the surgeon who is positioned to perform microsurgery. The accessibility in said microsurgical position thus implies that the second control panel which is arranged to display the second graphical user interface is arranged next to the treatment area, i.e. less than 0.5m away from field of surgery, preferentially less than 0.3m away from field of surgery.

In a preferred embodiment of such a laser assisted eye treatment system, the characterization unit comprises an observation unit, allowing a continuous observation of the eye treatment process. Advantageously, such an observation unit comprises an operation microscope system.

The operation microscope system may include a microscope head video camera for a continuous monitoring and transmission of the images and an adapter to fix the operation microscope system to the laser treatment unit, e.g. on laser applicator of the laser treatment unit.

Advantageously, the control panel of the second graphical user interface of the laser assisted eye treatment system comprises a small size screen. Further it is advantageous, if the control panel of the second graphical user interface is fixed adjacent to the observation unit.

Preferably, the size of the control panel screen arranged to display the second graphical user interface is equal to or smaller then13". This ensures that the control panel screen may be arranged close to the surgeons working place, but is not in way during surgery.

In a preferred embodiment, the second control panel arranged to display the second graphical user interface of the laser assisted eye treatment system is adapted to use by a surgeon in a sterile environment. Preferably, the second control panel is a touch screen control panel. A touch screen panel is much easier to clean and to protect from contamination than e.g. a keyboard or a mouse. For sterile operation, the second control panel can be operated through a sterile drape or using a sterile stylus.

As it is not necessary for the first control panel to be placed such that it is visible for the surgeon, the first control panel arranged to display the first graphical user interface may comprise a large size screen. It also may be placed at a distance to the microsurgical position.

Preferably the size of the first control panel screen is equal to or larger than 22" to ensure that all information necessary during e.g. the preparation steps are communicated on the screen in a clearly arranged way.

Placing the first control panel arranged to display the first graphical user interface at a distance may require making it accessible by a network connection. Remote access may be given to the first graphical user interface, e.g., to be able to plan an laser treatment of the eye also from outside the operation room.

In an advantageous embodiment of the laser assisted eye treatment system, the subset of information which the second graphical user interface is configured to communicate is defined dependent on a choice of an eye treatment process. To give an example: If the laser assisted eye treatment system is to be used only for cataract-related treatment (i.e. laser capsulotomy and fragmentation), the subset of information displayed on the second graphical user interface will be more restricted than if the laser assisted eye treatment system would be used for cataract surgery combined with a correction of astigmatism. The restriction of access to the communication of information of the second graphical user interface is thus dependent on the surgery option.

In a preferred embodiment of the laser assisted eye treatment system the system controller - and/or the first and second user graphical user interfaces - comprises predefined settings. These predefined settings may be categorized. The first and the second graphical user interfaces are then configured to select a setting out of the predefined settings according to the required information.

The predefined settings may be given in form of look-up tables, the surgeon or an assistant may scroll through and choose the best fitting setting.

It is of further advantage, if the predefined settings are categorized. A possible categorization would be e.g. the following: fixed information, surgeon dependent information, patient group dependent information, patient specific information. To give an example for patient group dependent information, there might be different levels of cataracts density to be chosen.

The selection of a setting out of the predefined settings may be possible in a completely automatic way or by requiring additional data to be entered manually.

The system controller of the laser assisted eye treatment system is preferentially encoded by an automatic routine for planning the eye treatment based on the definition of the anatomy of the eye. Preferentially, the definition of the anatomy of the eye is used for planning incisions in the eye: In accordance with this planning the focus of the pulsed laser beam is guided to the required positions in the eye to provoke photodisruption there. When reviewing the planning, even a possible correction of the incisions is done via the correction of the anatomy. The anatomy of the eye being directly visible, this is a safer way to plan the incisions than a direct incision planning.

As eye surgeons often practice until the age of retirement and the number of presbyopes is increasing with the age of the persons, in a preferred embodiment of the laser assisted eye treatment system the first graphical user interface of first control panel and/or the second graphical user interface of the second control panel of the are configured to display information with a high level of brightness.

This means that background and any larger spatial fields of information are displayed in white or other bright colors. This is advantageous as the bright display will cause the pupils of the user to constrict, thus increasing their depth of field. This is especially advantageous for presbyopes, making it easier to clearly see the display at near distance.

This feature of displaying information entirely using bright colors may, of course, also be used independently of the laser assisted eye treatment system for any display where a high depth of field is required and which are specially designed for presbyopes and in particular for such displays used in a difficult environment.

In a further advantageous embodiment of the laser assisted eye treatment system, the first graphical user interface of the first control panel and/or the second graphical user interface of the second control panel is configured to display composite images. Composite images may be representations of measured eye structures and/or representations of the generated surface which are superimposed to representations of the planned eye treatment, e.g., so as to observe the success of the laser treatment of the eye compared to the planned eye treatment.

It is further preferable, if different categories of the composite images are periodically superimposed, to have a better visibility of the laser effects.

In another advantageous embodiment of the laser assisted eye treatment system the images are arranged in an expand and collapse modus on the first graphical user interface of the first control panel display and/or on the second graphical user interface of the second control panel display. This allows changing the display mode between an overview mode and a detailed view mode. An image displayed on the control panel might e.g. also be a video image.

If the characterization unit scans are arranged in correct positional arrangement to an image on the first graphical user interface of the first control panel and/or on the second graphical user interface of the second control panel, this allows a better orientation for the surgeon. A characterization unit used to generate scans may be e.g. an optical coherence tomography (OCT) unit.

The problem is further solved by an eye treatment method using a laser beam for treating a patient's eye employing two graphical user interfaces. The methods disclosed herein are not part of the claimed invention and given for explanatory reasons only.

In detail, the problem is solved by an eye treatment method using a laser beam for treating a patient's eye following a workflow comprising the working steps of:
- selecting a patient
- planning the eye treatment,
- defining an anatomy of the patient's eye,
- reviewing the planning of the laser treatment, and
- applying a laser treatment to the eye.

In particular, the eye treatment method may comprise the use of the above described eye treatment system.

An eye treatment method as understood here is a method for treating eye tissue, which may for example be the cornea, the limbus, the pupil or the lens, including the lens capsular membrane. It also may be another material, including an artificial material in a patient's eye.

An eye treatment method using a laser beam may result in an effect of cutting, e.g. by photodisruption using a focused pulsed laser beam. It may result in an effect of "bonding" or another modification of the eye tissue by coagulation. Further it may result in a shaping effect using laser ablation.

The exemplary eye treatment method is characterized in that the information required for the working step of selecting a patient is communicated via a first graphical user interface. In this working step, it is possible either to select a concrete patient's name and thus to load all data registered for this patient, or to select an "anonymous patient" to start an eye treatment routine without loading personalized data.

Further, the information required for the working step of planning the eye treatment is communicated via a first and/or a second graphical user interface. The planning of the eye treatment is done on a virtual model eye. Last but not least, the information required for the working steps of defining the anatomy of the patient's eye, reviewing the planning and executing the laser treatment is communicated via a second graphical user interface. This second graphical user interface displayed on a second control panel may have an easy and comprehensible structure, as it serves only for particular steps requiring a lower amount and especially a lower variety of information to be communicated. That is why the second control panel may be smaller than the first control panel displaying the first graphical user interface. Therefore the second control panel displaying the second graphical user interface may be disposed next to the surgeon. Thus the surgeon can personally control the eye treatment using this second graphical user interface.

During the review of the planning, the planning of the eye treatment is adapted - if necessary - according to the results of the definition of the anatomy of the patients eye.

Preferentially the planning of the eye treatment is started in reference to an ideal anatomic model of the eye, and the step of defining an anatomy of the patient's eye is followed by an automatic pattern generation of the incisions based on the definition of the anatomy of the eye, starting from the eye treatment planning. If the surgeon feels that he or she should correct the incisions, he or she will do it by correcting the definition of the anatomy. In this embodiment, the surgeon thus does not shift the position of an incision but always intervenes on the definition of the anatomy, that automatically determines the position of the incisions. If the surgeon is satisfied, he only approves the proposed generated pattern of the incisions.

In an example of the method, the workflow of the eye treatment method further comprises the working steps of docking the patient's eye to a laser assisted eye treatment system, in general before defining an anatomy of the patient's eye, and undocking the patient's eye from the laser assisted eye treatment system, in general after the laser treatment, wherein the information required for these working steps is communicated via the second graphical user interface.

The workflow may further comprise the working step of reporting after the laser treatment, which is generally done after the undocking if the docking and undocking steps are done, wherein the information required is communicated via the first graphical user interface.

Finally the workflow of the eye treatment method may further comprise the working step of planning the eye treatment, e.g., incisions into an eye tissue or other modifications of an eye tissue by the laser beam, wherein the information required is communicated via the first and/or the second graphical user interface.

Preferentially, the eye treatment method makes use of a laser assisted eye treatment system as described above.

Therefore the system controller of the laser assisted eye treatment system may be encoded to carry out the described eye treatment method. Particularly, the system controller may be encoded to provide the relevant first and/or second graphical user interface to the first and/or second control panel for each of the working steps.

The laser assisted eye treatment system acording to the invention as well as the described exemplary eye treatment method allow a surgeon or other staff member to choose among quite different modes of an eye treatment, all of them operating safely and autonomously, especially during the critical sequences, proposing to the surgeon and other staff members a simple comprehensible and intuitive structure of a workflow.

The laser assisted eye treatment system according to the invention as well as the described exemplary eye treatment method are easily operable in a sterile environment, and actively avoid accidents arising from maloperation by the surgeon or another person handling the system.

As the workflow is clearly structured and the critical working steps as well as their content are reduced to the absolute minimum, it further helps to shorten a possible docking time of the eye to be treated to the laser assisted eye treatment system.

The object, features and advantages of the present invention and its modes of operation as well as advantageous combinations of different features will become more apparent considering the following **detailed description and embodiments** with reference to the accompanying drawings.
Fig. 1 shows the criticality as well the amount and variety of information to be communicated during an eye treatment as already mentioned above.
Fig. 2 shows a scheme of different features and their relationship of the laser assisted eye treatment system.
Fig. 3 shows possibilities of working steps repartitions as well as exemplary first and second control panel screens of the first and second graphical user interfaces.
Fig. 4 shows a first embodiment of a working steps repartition between first and second graphical user interface for a first eye treatment workflow.
Fig. 5 shows a second embodiment of a working steps repartition between first and second graphical user interface for a second eye treatment workflow.
Fig. 6 shows a third embodiment of a working steps repartition between first and second graphical user interface for a third eye treatment workflow.
Fig. 7 is a detailed view of the control panel screen of the second graphical user interface.

As already mentioned, fig. 1 shows the criticality as well the amount and variety of information to be communicated during an eye treatment the laser assisted eye treatment system 1 set-up is considering.

The planning of an eye treatment by a laser assisted eye treatment system 1 may be done at any time and also outside the operation room. It is requiring a large amount and variety of information to be communicated, but the information is not time critical and a given wrong information may be corrected.

The first part of the preparation of an eye treatment by a laser assisted eye treatment system 1 is generally done in the operation room, but not by the surgeon himself, but by an assistant. Even the preparation steps are not time critical and wrong information may still be corrected.

The final part of the preparation of an eye treatment by a laser assisted eye treatment system 1 is necessarily done in the operation room by the surgeon himself, just prior to the laser treatment. The communicated information are already critical, as there is no further "control instance" prior to the eye treatment.

Finally, information to be communicated during all eye treatment working steps, or, if the eye to be treated is docked to the laser assisted eye treatment system 1, all working steps with docked eye E, is very critical information. Wrong information can cause immediate and non-reversible damage of the eye E.

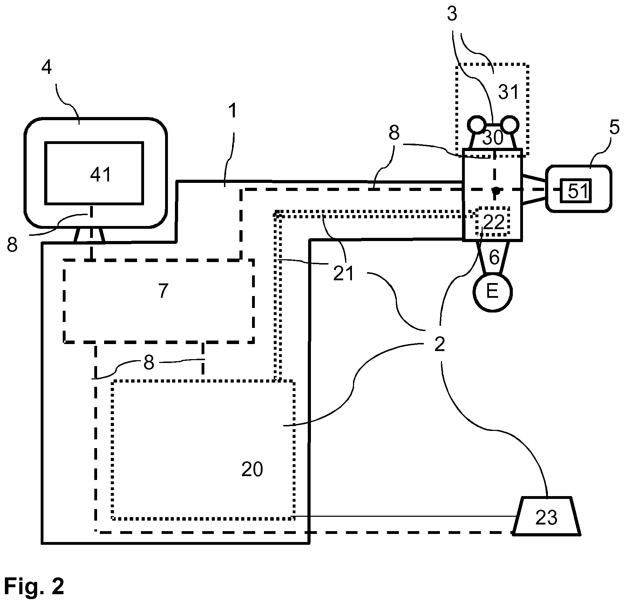

Fig. 2 shows a scheme of an example of a laser assisted eye treatment system 1 according to the invention, especially explaining its substructure and different features and the relationship between the different features.

A laser assisted eye treatment system 1 is configured to carry out an eye treatment workflow. It comprises a laser treatment unit 2, which includes a laser console 20, a laser guiding system 21, a laser applicator 22 and a laser foot switch 23. The laser console 20 also comprises the laser source, i.e. it generates a pulsed laser beam. For the example of fig. 2 it comprises a femtosecond laser source, generating a pulsed femtosecond laser beam.

The laser guiding system 21 deflects the laser beam in a flexible manner. It is movable for placing the laser applicator 22, which is following at the further end of the laser guiding system 21, at the required position over a patient's eye E. The patient, not shown in fig. 2, may be placed on an operation bed, in an horizontal position beside the laser assisted eye treatment system 1. The flexible laser guiding system 21, which allows to move the laser applicator 22 within a three-dimensional space along three directions, is then actuated to adjust to position of the laser applicator 22 exactly over the eye E of the patient. Alternatively, the patient may be placed in the position directly below the laser applicator 22.

Using a patient interface 6, the patient's eye may then be docked to the laser assisted eye treatment system 1.

The laser foot switch 23 of the example is used to command the action of the pulsed laser beam. Especially in case of emergency, such an additional user interface is very useful, as it offers the shortest way to interrupt the laser beam.

The laser assisted eye treatment system 1 further comprises a characterization unit 3. For the present example the characterization unit 3 comprises an operation microscope system 30 and an OCT (optical coherence tomography) system 31, which are not displayed in detail in fig. 2, allowing a permanent and detailed observation of the eye region to be treated.

The laser assisted eye treatment system 1 further comprises a first control panel 4 displaying a first graphical user interface 41.

A system controller 7 is configured to control the laser assisted eye treatment system 1 and to provide the first graphical user interface 41 to the first control panel 4 in an interactive way, taking into the account the inputs done by the surgeon or an assistant. The system controller 7 is coupled to the respective features of the laser assisted eye treatment system 1 via communication paths 8.

The laser assisted eye treatment system 1 further comprises a second control panel 5 displaying a second graphical user interface which is provided by the system controller 7 to this second control panel 5. This second graphical user interface 51 is configured to communicate a subset of information out of an entire set of information required for the eye treatment workflow, notably to communicate a subset used during the critical working steps of the eye treatment using the pulsed femtosecond laser beam to cut eye tissue in this case. The second control panel 5 displaying the second graphical user interface 51 is arranged near to the micro-surgery position, thus being easily accessible by the surgeon carrying out the critical working steps of the laser treatment of the patient's eye E.

The surgeon can thus use of the second graphical user interface, which displays the information of the subset in a clear and structured way, check results of an anatomy definition working step, which is a characterization working step, and intervene when he considers that a modification of the information is required.

Fig. 2 also gives an idea of the physical appearance of an eye treatment system for illustrating the local conditions for a laser assisted eye treatment system 1, comprising a laser treatment system 2 with a laser console 20, a laser guiding system 21 and a laser applicator 22 and a characterization unit 3 with an operation microscope 30.

As the patient has to be placed under the laser applicator 22 during the eye treatment, and in most cases the eye E has to be fixed to the laser assisted eye treatment system 1 by a patient interface 6, the laser treatment system 2 comprises flexible parts to adapt the position of the laser applicator 22 over the patient's eye E.

The surgeon being situated beside the patient makes it necessary to arrange the second control panel 5 displaying the second graphical user interface 51 on the moving parts, where only a small space is available to do so.

It is also visible that the large first control panel 4 displaying the first graphical user interface 41 is at a considerable distance from the surgeon, as the large first control panel 4 would not be possible to be fixed at the moving parts of the laser assisted eye treatment system 1.

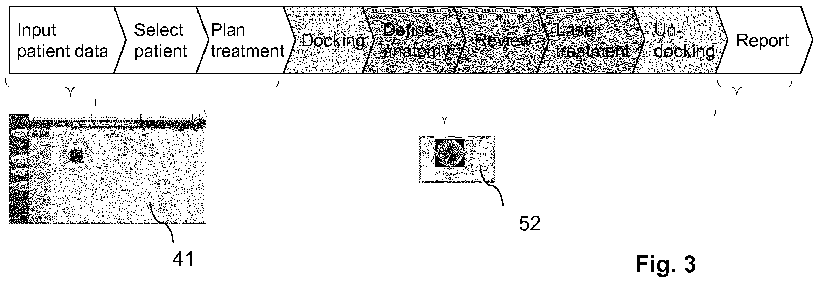

Fig. 3 shows possibilities of working steps repartitions between the first graphical user interface 41 and a second graphical user interface 51, as well as a first control panel screen 4 displaying an exemplary first graphical user interface 41 and a second control panel screen 5 displaying an exemplary second graphical user interface 51.

Within this exemplary working steps repartition, at least the working steps of defining anatomy, reviewing and laser treatment have to be done while docking the eye E to the laser assisted eye treatment system 1.

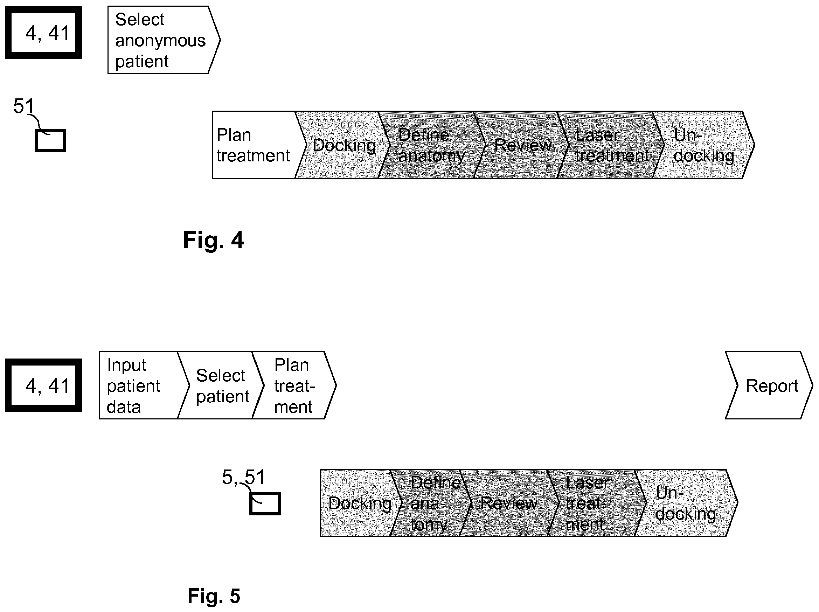

Fig. 4 shows a first embodiment of a working steps repartition between first 41 and second graphical user interface 51 for a first eye treatment workflow. It is an exemplary workflow for an eye treatment without entering or storing patient data. In this case it is possible for the surgeon to plan and perform the key functions of the entire laser procedure without entering patient data such as name, birthday, etc.

The surgeon or a staff member only creates or selects an anonymous patient and then presses "start procedure" on the large control panel screen 4 displaying the first graphical user interface 41.

Everything else - including treatment planning - can then be done on the small control panel screen 5 displaying the second graphical user interface 51. On the second graphical user interface 51 of this small screen 5, the surgeon thus plans the treatment, docks on the eye E, performs imaging, defines anatomy, does final review, and starts the laser.

Fig. 5 shows a second embodiment of a working steps repartition between first 41 and second graphical user interface 51 for a second eye treatment workflow. It is a workflow for an eye treatment storing patient data. The entering of the patient data - for one or more patients at once -, the selection of a patient and the planning of the treatment of the selected patient is done using the first graphical user interface 41 of the large control panel screen 4. This might be done by the surgeon or by a staff member based on instructions from the surgeon. Then the procedure is started on the first graphical user interface 41 of the large control panel screen 4.

All other steps are done by the surgeon using the second graphical user interface 51 of the small control panel screen 5 after the procedure has been started. On the second graphical user interface of the small control panel screen 5, the surgeon thus reviews the planning, docks on the eye E, performs the imaging, defines the anatomy, does a final review, and starts the laser for the laser treatment of the eye E, further observes and, if necessary, corrects the laser treatment.

A final report of the achieved laser treatment is then done after the undocking of the eye E, which was initiated on the second graphical user interface 51 of the small control panel screen 5, on the first graphical user interface 41 of the large control panel screen 4. This reporting may be done by the surgeon or by a staff member again based on the instructions from the surgeon.

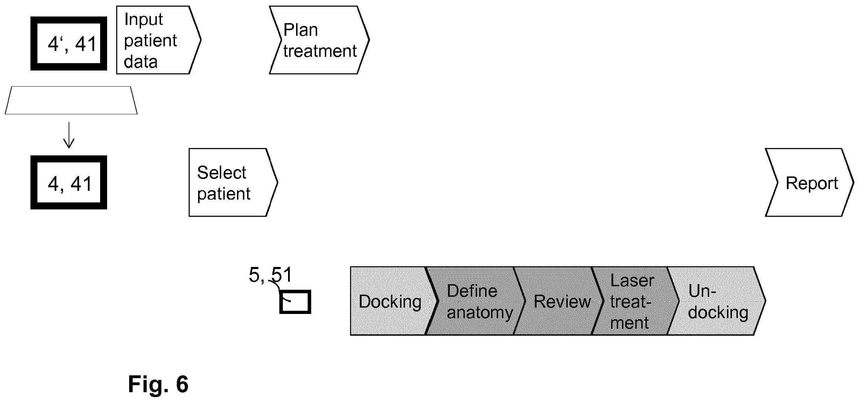

Fig. 6 shows a third embodiment of a working steps repartition between first 41 and second graphical user interface 51 for a third eye treatment workflow. It is another workflow for an eye treatment storing patient data.

In this case, the patient data may be entered and the procedure planned on a system located outside of the operation room - either by the surgeon or by a staff member based on instructions from the surgeon - on a network personal computer by remote access to the first graphical user interface 41 via a network connection.

The data is thus imported to the system in the operation room via the network connection. The surgeon or a staff member then selects the patient on the first graphical user interface of the large control panel screen 4 and presses "start procedure".

On the second graphical user interface of the small control panel screen 5, the surgeon reviews the planning, docks on the eye E, performs the imaging, defines the anatomy, does a final review, and starts the laser treatment of the eye E, further observes and, if necessary, corrects the laser treatment.

Again, a final report of the achieved laser treatment is then done on the first graphical user interface 41 of the large control panel screen 4 after the undocking of the eye E, which was initiated on the second graphical user interface 51 of the small control panel screen 5. This reporting may be done by the surgeon or by a staff member again based on the instructions from the surgeon.

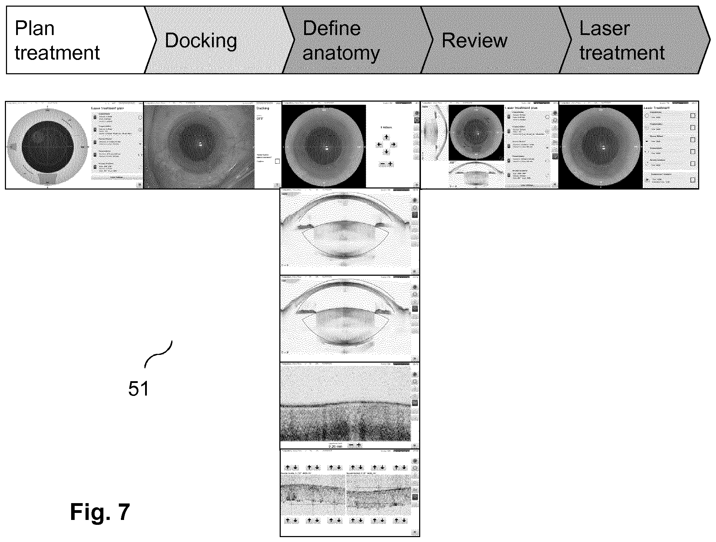

Fig. 7 is a detailed view of the second graphical user interface 51 of the second control panel screen 5. The figure illustrates the arrangement of images in an expand and collapse modus on this second graphical user interface of the second control panel screen 5. The figure illustrates as well the correct positional arrangement of OCT unit scans to an image on the second control panel screen 5. The OCT unit scans were done for characterization of the anatomy of the eye. This can be seen in particular in the review image.

The features of the invention mentioned above and explained in several embodiments, are not only applicable in the combinations explained in the exemplary embodiments, but also in other combinations or alone without exceeding the scope of the present invention.

A feature related to and characterized for the system applies in analogy to the relevant method, while method features may be applied as functional features of the system described accordingly.

## Claims

1. A laser assisted eye treatment system (1) for carrying out an eye treatment workflow, comprising:
- a laser treatment unit (2, 20, 21, 22, 23) with a laser source generating a pulsed laser beam,
- a characterization unit (3),
- a system controller (7), configured to control the laser assisted eye treatment system (1),
- a first control panel (4) arranged to display a first graphical user interface (41) provided by the system controller (7) and configured to communicate information required for the eye treatment workflow,
- a second control panel (5) arranged to display a second graphical user interface (51) provided by the system controller (7) and
- wherein the second control panel (5) is configured to communicate a subset of information, being a restricted selection of information, directly available to a surgeon and used during the laser treatment by the surgeon, out of an entire set of information required for the eye treatment workflow.

2. The laser assisted eye treatment system (1) according to claim 1, wherein the first graphical user interface (41) is configured to communicate a first subset of information and the second graphical user interface (51) is configured to communicate a second subset of information out of the entire set of information required for the workflow, the first and the second subset of information being non-overlapping or partly overlapping subsets.

3. The laser assisted eye treatment system (1) according to claim 1 or 2, wherein the eye treatment workflow comprises several working steps corresponding to different stages of an eye treatment process and wherein each of the working steps is assigned to the first (41) and/or to the second graphical user interface (51).

4. The laser assisted eye treatment system (1) according to one of claims 1 to 3, further comprising a docking unit (6) arranged for establishing a defined relationship between the laser assisted eye treatment system (1) and a patient's eye (E) to be treated.

5. The laser assisted eye treatment system (1) according to one of claims 1 to 4, wherein the second control panel (5) is accessible in a microsurgical position.

6. The laser assisted eye treatment system (1) according to one of claims 1 to 5, wherein the characterization unit (3) comprises an observation unit, especially comprising an operation microscope system (30).

7. The laser assisted eye treatment system (1) according to claim 6, wherein the second control panel (5) comprises a small size screen, preferably equal to or smaller then13", and/or is fixed adjacent to the observation unit.

8. The laser assisted eye treatment system (1) according to one of claims 1 to 7, wherein the second control panel (5) is adapted to use by a surgeon in a sterile environment, the second control panel (5) preferably being a touch screen control panel.

9. The laser assisted eye treatment system (1) according to one of claims 1 to 8, wherein the first control panel (4) comprises a large size screen, preferably equal to or larger than 22", and/or is placed at distance to the microsurgical position.

10. The laser assisted eye treatment system (1) according to one of claims 1 to 9, wherein the subset of information which the second graphical user interface (51) is configured to communicate, is defined dependent on a choice of an eye treatment process.

11. The laser assisted eye treatment system (1) according to one of claims 1 to 10, wherein the system controller (7) comprises predefined settings, the predefined settings preferably being categorized, and wherein the first (41) and second graphical user interfaces (51) are configured to be provided with a selected setting out of the predefined settings according to the required information.

12. The laser assisted eye treatment system (1) according to one of claims 1 to 11, wherein the system controller (7) is encoded by an automatic routine for planning the eye treatment, preferentially for planning incisions into the eye (E), based on the definition of the anatomy of the eye (E).

13. The laser assisted eye treatment system (1) according to one of claims 1 to 12, wherein the first graphical user interface (41) of the first control panel (4) and/or the second graphical user interface (51) of the second control panel (5) is configured to display information at a high level of brightness by displaying background and larger spatial fields of information in white or other bright colors.

14. The laser assisted eye treatment system (1) according to one of claims 1 to 13, wherein first graphical user interface (41) of the first control panel (4) and/or the second graphical user interface (51) of the second control panel (5) is configured to display composite images and wherein different categories of the composite images are preferably periodically superimposed.

15. The laser assisted eye treatment system (1) according to one of claims 1 to 14, wherein images are arranged in an expand and collapse modus on the first graphical user interface (41) of the first control panel (4) and/or on the second graphical user interface (51) of the second control panel (5) and/or wherein characterization unit scans are arranged in correct positional arrangement to an image on the first graphical user interface (41) of the first control panel (4) and/or the second graphical user interface (51) of the second control panel (5).

## Patentansprüche

1. Laserunterstütztes Augenbehandlungssystem (1) zum Durchführen eines Augenbehandlungsarbeitsablaufs, umfassend:
- eine Laserbehandlungseinheit (2, 20, 21, 22, 23) mit einer Laserquelle, die einen gepulsten Laserstrahl erzeugt,
- eine Charakterisierungseinheit (3),
- eine Systemsteuerung (7), die ausgestaltet ist, das laserunterstützte Augenbehandlungssystem (1) zu steuern,
- ein erstes Steuer-Panel (4), das eingerichtet ist, eine erste graphische Benutzerschnittstelle (41) anzuzeigen, die von der Systemsteuerung (7) bereitgestellt wird und ausgestaltet ist, Informationen zu kommunizieren, die für den Augenbehandlungsarbeitsablauf erforderlich sind,
- ein zweites Steuer-Panel (5), das eingerichtet ist, eine zweite graphische Benutzerschnittstelle (51) anzuzeigen, die durch die Systemsteuerung (7) bereitgestellt wird, und wobei
das zweite Steuer-Panel (5) ausgestaltet ist, einen Teilsatz der Informationen, der eine eingeschränkte Auswahl der Informationen ist, die einem Chirurgen direkt zur Verfügung steht und während der Laserbehandlung durch den Chirurgen verwendet wird, aus einem vollständigen Satz von Informationen, der für den Augenbehandlungsarbeitsablauf erforderlich ist, zu kommunizieren.

2. Laserunterstütztes Augenbehandlungssystem (1) nach Anspruch 1, wobei die erste graphische Benutzerschnittstelle (41) eingerichtet ist, einen ersten Teilsatz der Informationen zu kommunizieren, und die zweite graphische Benutzerschnittstelle (51) eingerichtet ist, einen zweiten Teilsatz an Informationen aus dem vollständigen Satz der Informationen, die für den Arbeitsablauf erforderlich sind, zu kommunizieren, wobei der erste Teilsatz und der zweite Teilsatz der Informationen nicht überlappende oder teilweise überlappende Teilsätze sind.

3. Laserunterstütztes Augenbehandlungssystem (1) nach Anspruch 1 oder 2, wobei der Augenbehandlungsarbeitsablauf mehrere Arbeitsschritte umfasst, die unterschiedlichen Stadien eines Augenbehandlungsprozesses entsprechen, und wobei jeder der Arbeitsschritte der ersten (41) und/oder der zweiten graphischen Benutzerschnittstelle (51) zugewiesen ist.

4. Laserunterstütztes Augenbehandlungssystem (1) nach einem der Ansprüche 1 bis 3, des Weiteren umfassend eine Docking-Einheit (6), die angeordnet ist, um eine definierte Beziehung zwischen dem laserunterstützten Augenbehandlungssystem (1) und einem zu behandelnden Patientenauge (E) aufzubauen.

5. Laserunterstütztes Augenbehandlungssystem (1) nach einem der Ansprüche 1 bis 4, wobei das zweite Steuer-Panel (5) in einer mikrochirurgischen Position zugänglich ist.

6. Laserunterstütztes Augenbehandlungssystem (1) nach einem der Ansprüche 1 bis 5, wobei die Charakterisierungseinheit (3) eine Beobachtungseinheit, insbesondere ein Operationsmikroskop-System (30) umfasst.

7. Laserunterstütztes Augenbehandlungssystem (1) nach Anspruch 6, wobei das zweite Steuer-Panel (5) einen kleinen Bildschirm umfasst, vorzugsweise gleich oder kleiner als 13", und/oder benachbart zu der Beobachtungseinheit fixiert ist.

8. Laserunterstütztes Augenbehandlungssystem (1) nach einem der Ansprüche 1 bis 7, wobei das zweite Steuer-Panel (5) eingerichtet ist, durch einen Chirurgen in einer sterilen Umgebung verwendet zu werden, wobei das zweite Steuer-Panel (5) vorzugsweise ein Touchscreen-Steuer-Panel ist.

9. Laserunterstütztes Augenbehandlungssystem (1) nach einem der Ansprüche 1 bis 8, wobei das erste Steuer-Panel (4) einen großen Bildschirm umfasst, vorzugsweise gleich oder größer als 22", und/oder in einem Abstand zu der mikrochirurgischen Position platziert ist.

10. Laserunterstütztes Augenbehandlungssystem (1) nach einem der Ansprüche 1 bis 9, wobei der Teilsatz der Informationen, den die zweite graphische Benutzerschnittstelle (51) konfigurationsgemäß kommunizieren soll, in Abhängigkeit von einer Auswahl eines Augenbehandlungsverfahrens definiert ist.

11. Laserunterstütztes Augenbehandlungssystem (1) nach einem der Ansprüche 1 bis 10, wobei die Systemsteuerung (7) vordefinierte Einstellungen umfasst, die vordefinierten Einstellungen vorzugsweise kategorisiert sind, und wobei die erste (41) und die zweite graphische Benutzerschnittstelle (51) eingerichtet sind, mit einer ausgewählten Einstellung aus den vordefinierten Einstellungen entsprechend den erforderlichen Informationen bereitgestellt zu werden.

12. Laserunterstütztes Augenbehandlungssystem (1) nach einem der Ansprüche 1 bis 11, wobei die Systemsteuerung (7) durch eine automatische Routine für die Planung der Augenbehandlung kodiert wird, vorzugsweise für die Planung von Inzisionen in das Auge (E), basierend auf der Definition der Anatomie des Auges (E).

13. Laserunterstütztes Augenbehandlungssystem (1) nach einem der Ansprüche 1 bis 12, wobei die erste graphische Benutzerschnittstelle (41) des ersten Steuer-Panels (4) und/oder die zweite graphische Benutzerschnittstelle (51) des zweiten Steuer-Panels (5) eingerichtet ist/sind, Informationen mit einem hohen Helligkeitsniveau anzuzeigen, indem Hintergrund und größere räumliche Informationsfelder in weiß oder anderen hellen Farben angezeigt werden.

14. Laserunterstütztes Augenbehandlungssystem (1) nach einem der Ansprüche 1 bis 13, wobei die erste graphische Benutzerschnittstelle (41) des ersten Steuer-Panels (4) und/oder die zweite graphische Benutzerschnittstelle (51) des zweiten Steuer-Panels (5) eingerichtet ist/sind, zusammengesetzte Bilder anzuzeigen, und wobei unterschiedliche Kategorien der zusammengesetzten Bilder vorzugsweise periodisch überlagert werden.

15. Laserunterstütztes Augenbehandlungssystem (1) nach einem der Ansprüche 1 bis 14, wobei Bilder in einem ausgeklappten und eingeklappten Modus auf der ersten graphischen Benutzerschnittstelle (41) des ersten Steuer-Panels (4) und/oder auf der zweiten graphischen Benutzerschnittstelle (51) des zweiten Steuer-Panels (5) angeordnet sind, und/oder wobei Scans der Charakterisierungseinheit in korrekter Positionsanordnung zu einem Bild auf der ersten graphischen Benutzerschnittstelle (41) des ersten Steuer-Panels (4) und/oder der zweiten graphischen Benutzerschnittstelle (51) des zweiten Steuer-Panels (5) angeordnet sind.

## Revendications

1. Système de traitement oculaire assisté par laser (1) pour effectuer un flux de travail de traitement oculaire, comprenant :
- une unité de traitement laser (2, 20, 21, 22, 23) dotée d'une source laser générant un faisceau laser pulsé,
- une unité de caractérisation (3),
- un dispositif de commande de système (7), configuré pour commander le système de traitement oculaire assisté par laser (1),
- un premier panneau de commande (4) conçu pour afficher une première interface utilisateur graphique (41) fournie par le dispositif de commande de système (7) et configurée pour communiquer les informations nécessaires au déroulement du flux de travail de traitement oculaire,
- un second panneau de commande (5) conçu pour afficher une seconde interface utilisateur graphique (51) fournie par le dispositif de commande de système (7), et
le second panneau de commande (5) étant configuré pour communiquer un sous-ensemble d'informations, qui est une sélection restreinte d'informations, directement accessible à un chirurgien et utilisée pendant le traitement laser par le chirurgien, parmi un ensemble complet d'informations requis pour le flux de travail de traitement oculaire.

2. Système de traitement oculaire assisté par laser (1) selon la revendication 1, la première interface utilisateur graphique (41) étant configurée pour communiquer un premier sous-ensemble d'informations et la seconde interface utilisateur graphique (51) étant configurée pour communiquer un second sous-ensemble d'informations parmi l'ensemble complet d'informations requis pour le flux de travail, le premier et le second sous-ensemble d'informations étant des sous-ensembles qui ne se chevauchent pas ou qui se chevauchent partiellement.

3. Système de traitement oculaire assisté par laser (1) selon la revendication 1 ou 2, le flux de travail de traitement oculaire comprenant plusieurs étapes de travail correspondant à différents stades d'un processus de traitement oculaire et chacune des étapes de travail étant attribuée à la première (41) et/ou à la seconde interface utilisateur graphique (51).

4. Système de traitement oculaire assisté par laser (1) selon l'une des revendications 1 à 3, comprenant en outre une unité de raccordement (6) conçue pour établir une relation définie entre le système de traitement oculaire assisté par laser (1) et l'œil d'un patient (E) à traiter.

5. Système de traitement oculaire assisté par laser (1) selon l'une des revendications 1 à 4, le second panneau de commande (5) étant accessible en position microchirurgicale.

6. Système de traitement oculaire assisté par laser (1) selon l'une des revendications 1 à 5, l'unité de caractérisation (3) comprenant une unité d'observation, comprenant notamment un système de microscope opératoire (30).

7. Système de traitement oculaire assisté par laser (1) selon la revendication 6, le second panneau de commande (5) comprenant un écran de petite taille, de préférence égal ou inférieur à 13", et/ou étant fixé de manière adjacente à l'unité d'observation.

8. Système de traitement oculaire assisté par laser (1) selon l'une des revendications 1 à 7, le second panneau de commande (5) étant adapté à l'utilisation par un chirurgien dans un environnement stérile, le second panneau de commande (5) étant de préférence un panneau de commande à écran tactile.

9. Système de traitement oculaire assisté par laser (1) selon l'une des revendications 1 à 8, le premier panneau de commande (4) comprenant un écran de grande taille, de préférence égal ou supérieur à 22", et/ou étant placé à distance de la position microchirurgicale.

10. Système de traitement oculaire assisté par laser (1) selon l'une des revendications 1 à 9, le sous-ensemble d'informations que la seconde interface utilisateur graphique (51) est configurée pour communiquer étant défini en fonction du choix d'un processus de traitement oculaire.

11. Système de traitement oculaire assisté par laser (1) selon l'une des revendications 1 à 10, le dispositif de commande de système (7) comprenant des réglages prédéfinis, les réglages prédéfinis étant de préférence catégorisés, et les première (41) et seconde interfaces utilisateurs graphiques (51) étant configurées pour être fournies avec un réglage sélectionné parmi les réglages prédéfinis en fonction des informations requises.

12. Système de traitement oculaire assisté par laser (1) selon l'une des revendications 1 à 11, le dispositif de commande de système (7) étant codé par une routine automatique pour planifier le traitement oculaire, de préférence pour planifier les incisions dans l'œil (E), sur la base de la définition de l'anatomie de l'œil (E).

13. Système de traitement oculaire assisté par laser (1) selon l'une des revendications 1 à 12, la première interface utilisateur graphique (41) du premier panneau de commande (4) et/ou la seconde interface utilisateur graphique (51) du second panneau de commande (5) étant configurées pour afficher des informations à un niveau élevé de luminosité en affichant l'arrière-plan et des champs spatiaux d'informations plus larges en blanc ou dans d'autres couleurs vives.

14. Système de traitement oculaire assisté par laser (1) selon l'une des revendications 1 à 13, la première interface utilisateur graphique (41) du premier panneau de commande (4) et/ou la seconde interface utilisateur graphique (51) du second panneau de commande (5) étant configurées pour afficher des images composites et différentes catégories d'images composites étant de préférence périodiquement superposées.

15. Système de traitement oculaire assisté par laser (1) selon l'une des revendications 1 à 14, les images étant arrangées selon un mode d'expansion et de réduction sur la première interface utilisateur graphique (41) du premier panneau de commande (4) et/ou sur la seconde interface utilisateur graphique (51) du second panneau de commande (5) et/ou des balayages de l'unité de caractérisation étant arrangés dans une position correcte par rapport à une image sur la première interface utilisateur graphique (41) du premier panneau de commande (4) et/ou sur la seconde interface utilisateur graphique (51) du second panneau de commande (5).
